# EUROPEAN PATENT SPECIFICATION

(11) **EP 2 192 865 B1**
(45) Date of publication and mention of the grant of the patent: **20.03.2019**
(21) Application number: 08780034.8
(22) Date of filing: 08.07.2008
(51) Int. Cl.: A61B 17/70, A61F 2/44

(54) **INTERSPINOUS SPACER**
INTERSPINÖSES ABSTANDSGLIED
ESPACEUR INTERÉPINEUX

(30) Priority: 09.07.2007 US 958876 P
(43) Date of publication of application: 09.06.2010
(73) Proprietor: Vertiflex, Inc., San Clemente, CA 92673 (US)
(72) Inventor: ALTARAC, Moti, Irvine, CA 92603 (US); TEBBE, Shawn, Dromiskin, Co. Louth (IE); REGLOS, Joey, Camia, Lake Forest, CA 92630 (US); CHENG, Yang, Foothill Ranch, CA 92610 (US); KADABA, Murali, P., Emerald Hills, CA 94062 (US); KIM, Daniel, H., Houston, TX 77007 (US)
(74) Representative: Donald, Jenny Susan
(86) International application number: PCT/US2008/008382
(87) International publication number: WO 2009/009049

(56) References cited:
- WO-A1-2005/009300
- WO-A2-2007/075788
- US-A1- 2004 172 135
- US-A1- 2004 181 282
- US-A1- 2006 264 938
- US-A1- 2007 032 790
- US-B1- 6 235 030
- US-B2- 7 083 649

## Description

### FIELD

The present invention generally relates to medical devices, in particular, implants for placement between adjacent spinous processes of a patient's spine.

### BACKGROUND

With spinal stenosis, the spinal canal narrows and pinches the spinal cord and nerves, causing pain in the back and legs. Typically, with age, a person's ligaments may thicken, intervertebral discs may deteriorate and facet joints may break down-all contributing to the condition of the spine characterized by a narrowing of the spinal canal. Injury, heredity, arthritis, changes in blood flow and other causes may also contribute to spinal stenosis.

Doctors have been at the forefront with various treatments of the spine including medications, surgical techniques and implantable devices that alleviate and substantially reduce debilitating pain associated with the back. In one surgical technique, a spacer is implanted between adjacent interspinous processes of a patient's spine. The implanted spacer opens the spinal canal, maintains the desired distance between vertebral body segments, and as a result, avoids impingement of nerves and relieves pain. For suitable candidates, an implantable interspinous spacer may provide significant benefits in terms of pain relief.

US2007032790 discloses a device for treating spinal stenosis having an implant body structure sized and configured to be positioned between the spinous processes of two adjacent vertebrae. The device may have a body portion having a first end portion, a second end portion and a sleeve between the first and second end portions. The device may also have at least two retainers positioned in and extendable from the body portion. A mechanism positioned within the body portion may be used to move the retainers between a retracted position and a deployed position. When the retainers are in the deployed position, the retainers may be positioned around the spinous process of at least one of two adjacent vertebrae.

Any surgery is an ordeal. However, the type of device and how it is implanted has an impact. For example, one consideration when performing surgery to implant an interspinous spacer is the size of the incision that is required to allow introduction of the device. Small incisions and minimally invasive techniques are generally preferred as they affect less tissue and result in speedier recovery times. As such, there is a need for interspinous spacers that work well with surgical techniques that are minimally invasive for the patient. The present invention sets forth such a spacer.

### SUMMARY

According to one aspect of the invention, an implantable spacer for placement between adjacent spinous processes is disclosed. The implant includes a body defining a longitudinal axis and passageway through at least a portion of the body. A first arm and a second arm are both connected to the body and capable of rotation with respect to the body. Each arm defines a configuration suitable for cradling a spinous process. Each arm has a proximal caming surface. The spacer further includes an actuator assembly connected to the body. The actuator assembly comprises an actuator having at least one bearing surface and a threaded shaft connected to the actuator and configured for movement with respect to the body. The actuator assembly is configured such that the actuator is disposed inside the body and configured to move relative to the body such that the at least one bearing surface contacts the caming surfaces of the arms to thereby rotate the arms from an undeployed configuration in which the arms are substantially parallel to the longitudinal axis of the body to a deployed configuration in which the arms are substantially perpendicular to the longitudinal axis of the body. The arms seat adjacent spinous processes when in the deployed configuration.

In an example not claimed, an implantable spacer for placement between adjacent spinous processes is disclosed. The spacer includes a body defining a longitudinal axis and passageway through at least a portion of the body. At least a first arm is connected to the body and capable of movement with respect to the body and configured for receiving a spinous process. The spacer further includes an actuator connected to the body, disposed inside the longitudinal passageway and configured to move relative to the body to deploy the at least first arm from an undeployed configuration in which the first arm is substantially parallel to the longitudinal axis of the body to at least one deployed configuration in which the first arm is substantially perpendicular to the longitudinal axis of the body. The first arm receives a spinous process when in the at least one deployed configuration.

According to another aspect of the invention, an implantable spacer for placement between adjacent spinous processes is disclosed. The spacer includes a body defining a longitudinal axis and passageway through at least a portion of the body. A first arm and a second arm are connected to the body and capable of rotation with respect to the body. Each arm has a pair of extensions and a saddle defining a configuration for seating a spinous process therein. Each arm has a proximal caming surface. The spacer further includes an actuator connected to the body and configured to move relative to the body to deploy the arms from an undeployed configuration in which the arms are substantially parallel to the longitudinal axis of the body to a deployed configuration in which the arms are substantially perpendicular to the longitudinal axis of the body. The arms seat adjacent spinous processes when in the deployed configuration. The spacer further includes a retainer configured to retain the actuator inside the body while permitting the actuator relative movement with respect to the body.

In another example not claimed, an implantable spacer for placement between a superior spinous process and an adjacent inferior spinous process is disclosed. The spacer includes a body defining a longitudinal axis and passageway through at least a portion of the body. At least a first arm is connected to the body and capable of movement with respect to the body. The first arm has a configuration for seating the superior spinous process. The spacer further includes an actuator connected to the body, disposed inside the longitudinal passageway and configured to move relative to the body to deploy the at least first arm from an undeployed configuration in which the first arm is substantially parallel to the longitudinal axis of the body to at least one deployed configuration in which the first arm is substantially perpendicular to the longitudinal axis of the body. The first arm seats the superior spinous process when in the at least one deployed configuration.

A method, not claimed for implanting an interpsinous spacer into a patient is disclosed. The method includes the step of providing an implantable spacer configured for placement between adjacent spinous processes. The spacer includes a body having a longitudinal axis. The spacer further includes at least a first arm connected to the body and capable of movement with respect to the body and configured for receiving a spinous process. The spacer further includes an actuator connected to the body and configured to move relative to the body to deploy the at least first arm from an undeployed configuration in which the first arm is substantially parallel to the longitudinal axis of the body to at least one deployed configuration in which the first arm is substantially perpendicular to the longitudinal axis of the body. The first arm receives a spinous process when in the at least one deployed configuration. An incision is created in the patient. The spacer is inserted through the incision to a targeted interspinous process space between adjacent spinous processes. The actuator is moved to move the at least first arm from an undeployed configuration to a first deployed configuration. The actuator is moved to move the at least first arm from the first deployed configuration to a second deployed configuration.

### BRIEF DESCRIPTION OF THE DRAWINGS

The invention is best understood from the following detailed description when read in conjunction with the accompanying drawings. It is emphasized that, according to common practice, the various features of the drawings are not to-scale. On the contrary, the dimensions of the various features are arbitrarily expanded or reduced for clarity.
FIG. 1a illustrates a perspective view of a spacer according to the present invention.
FIG. 1b illustrates a side view of a spacer according to the present invention.
FIG. 1c illustrates a top view of a spacer according to the present invention.
FIG. 1d illustrates a cross-sectional view of the spacer of FIG. 1c taken along line A-A according to the present invention.
FIG. 1e illustrates an end view of a spacer according to the present invention.
FIG. 1f illustrates an exploded perspective view of a spacer according to the present invention.
FIG. 2a illustrates a perspective view of half of a body of a spacer according to the present invention.
FIG. 2b illustrates a side view of half of a body of a spacer according to the present invention.
FIG. 2c illustrates a perspective view of another half of a body of a spacer according to the present invention.
FIG. 2d illustrates a side view of the other half of a body of a spacer according to the present invention.
FIG. 3a illustrates a perspective view of a superior arm of a spacer according to the present invention.
FIG. 3b illustrates a back view of a superior arm of a spacer according to the present invention.
FIG. 3c illustrates a side view of a superior arm of a spacer according to the present invention.
FIG. 3d illustrates a perspective view of an inferior arm of a spacer according to the present invention.
FIG. 3e illustrates a back view of an inferior arm of a spacer according to the present invention.
FIG. 3f illustrates a side view of an inferior arm of a spacer according to the present invention.
FIG. 4a illustrates a perspective view of a half of a body and actuator assembly of a spacer according to the present invention.
FIG. 4b illustrates a side view of a portion of a half of a body and actuator assembly of a spacer according to the present invention.
FIG. 5a illustrates a side view of a spacer in a closed, undeployed configuration according to the present invention.
FIG. 5b illustrates a side view of a spacer in a partially deployed configuration according to the present invention.
FIG. 5c illustrates a side view of a spacer in a deployed configuration according to the present invention.
FIG. 5d illustrates a side view of a spacer in a deployed and extended configuration according to the present invention.
FIG. 6a illustrates a side, cross-sectional view of a spacer in a partially deployed configuration according to the present invention.
FIG. 6b illustrates a side, cross-sectional view of a spacer in a deployed configuration according to the present invention.
FIG. 6c illustrates a side, cross-sectional view of a spacer in a deployed and extended configuration according to the present invention.
FIG. 7a illustrates a side, semi-transparent view of a spacer in a partially deployed configuration according to the present invention.
FIG. 7b illustrates a side, semi-transparent view of a spacer in a deployed configuration according to the present invention.
FIG. 7c illustrates a side, semi-transparent view of a spacer in a deployed and extended configuration according to the present invention.
FIG. 8a illustrates a perspective view of a spacer according to the present invention.
FIG. 8b illustrates a side view of a spacer according to the present invention.
FIG. 8c illustrates a top view of a spacer according to the present invention.
FIG. 8d illustrates a cross-sectional view of the spacer of FIG. 8c taken along line A-A according to the present invention.
FIG. 8e illustrates an end view of a spacer according to the present invention.
FIG. 8f illustrates an exploded perspective view of a spacer according to the present invention.
FIG. 9a illustrates a perspective view of half of a body of a spacer according to the present invention.
FIG. 9b illustrates a side view of half of a body of a spacer according to the present invention.
FIG. 10a illustrates a perspective view of a superior arm of a spacer according to the present invention.
FIG. 10b illustrates a back view of a superior arm of a spacer according to the present invention.
FIG. 10c illustrates a side view of a superior arm of a spacer according to the present invention.
FIG. 10d illustrates a perspective view of an inferior arm of a spacer according to the present invention.
FIG. 10e illustrates a back view of an inferior arm of a spacer according to the present invention.
FIG. 10f illustrates a side view of an inferior arm of a spacer according to the present invention.
FIG. 11a illustrates a perspective view of a half of an actuator shaft and retainer of a spacer according to the present invention.
FIG. 11b illustrates a side view of a portion of an actuator shaft and retainer of a spacer according to the present invention.
FIG. 12a illustrates a side view of a spacer in a closed, undeployed configuration according to the present invention.
FIG. 12b illustrates a side view of a spacer in a partially deployed configuration according to the present invention.
FIG. 12c illustrates a side view of a spacer in a deployed configuration according to the present invention.
FIG. 12d illustrates a side view of a spacer in a deployed and extended configuration according to the present invention
FIG. 13a illustrates a side, cross-sectional view of a spacer in a partially deployed configuration according to the present invention.
FIG. 13b illustrates a side, cross-sectional view of a spacer in a deployed configuration according to the present invention.
FIG. 13c illustrates a side, cross-sectional view of a spacer in a deployed and extended configuration according to the present invention.
FIG. 14a illustrates a side, semi-transparent view of a spacer in a partially deployed configuration according to the present invention.
FIG. 14b illustrates a side, semi-transparent view of a spacer in a deployed configuration according to the present invention.
FIG. 14c illustrates a side, semi-transparent view of a spacer in a deployed and extended configuration according to the present invention.
FIG. 15a illustrates a side view of an insertion instrument connected to a spacer in a closed, undeployed configuration according to the present invention.
FIG. 15b illustrates a side view of an insertion instrument connected to a spacer in a partially deployed configuration according to the present invention.
FIG. 15c illustrates a side view of an insertion instrument connected to a spacer in a deployed configuration according to the present invention.
FIG. 15d illustrates a side view of an insertion instrument connected to a spacer in a deployed and extended configuration according to the present invention.
FIG. 16 illustrates a spacer according to the present invention deployed in an interspinous process space between two vertebral bodies and a supraspinous ligament.

### DETAILED DESCRIPTION

Before the subject devices, systems and methods are described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Unless defined otherwise, all technical and scientific terms used herein have the same meaning as commonly understood by one of ordinary skill in the art to which this invention belongs.

It must be noted that as used herein and in the appended claims, the singular forms "a", "an", and "the" include plural referents unless the context clearly dictates otherwise. Thus, for example, reference to "a spinal segment" may include a plurality of such spinal segments and reference to "the screw" includes reference to one or more screws and equivalents thereof known to those skilled in the art, and so forth.

The present invention is described in the accompanying figures and text as understood by a person having ordinary skill in the field of spinal implants and implant delivery instrumentation.

The present invention relates to an implantable spaces for placement between adjacent spinous processes as claimed hereafter. Preferred embodiments of the invention are set forth in the dependent claims. Associated methods are also described herein to aid understanding of the invention, but these do not form part of the claimed invention.

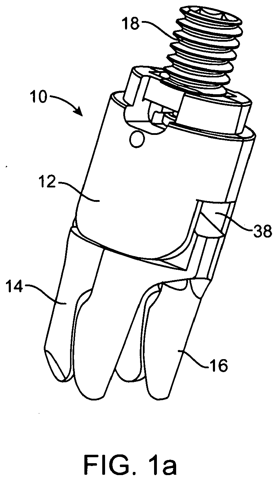

With reference to FIGs. 1a-1f, various views of a spacer 10 according to the present invention are shown. The spacer 10 includes a body 12 connected to a superior extension member or arm 14, an inferior extension member or arm 16, and an actuator assembly 18.

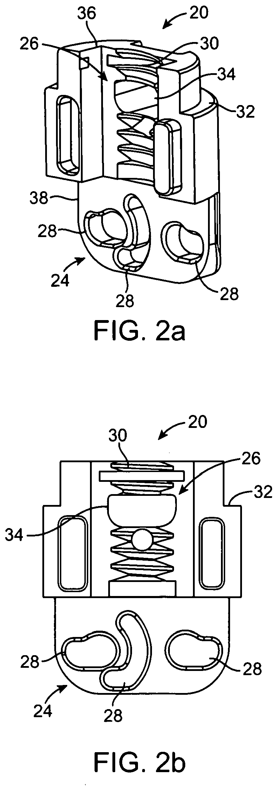

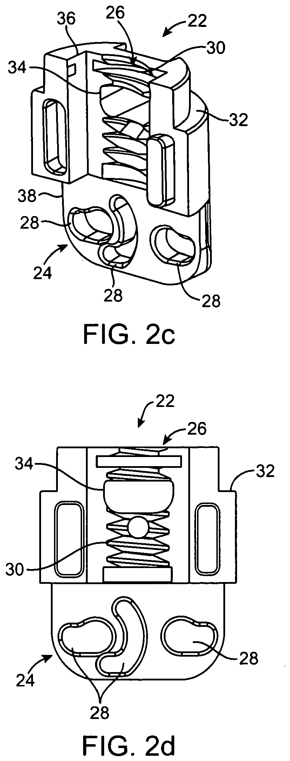

Turning now to FIGs. 2a-2d, the body 12 will now be described. The body 12 is shown to have a clamshell construction with a left body piece 20 (shown in FIGs. 2a and 2b) joined to a right body piece 22 (shown in FIGs. 2c and 2d) to capture arms 14, 16 inside. With the right and left body pieces 20, 22 joined together, the body 12 is generally cylindrical. It has a cross-sectional size and shape that allows for implantation between adjacent spinous processes and facilitates delivery into a patient through a narrow port or cannula.

The inside of the body 12 defines an arm receiving portion 24 and an actuator assembly receiving portion 26 with features formed in each of the left and right body pieces 20, 22 that together define the arm and actuator assembly receiving portions 24, 26. In one variation, the arm receiving portion 24 includes slots or openings 28 that receive pins formed on the arms 14, 16 such that the pins rotate and/or translate inside the openings 28. The actuator assembly receiving portion 26 includes a threaded passageway 30. Other features include a tongue and groove for mating with the opposite clamshell.

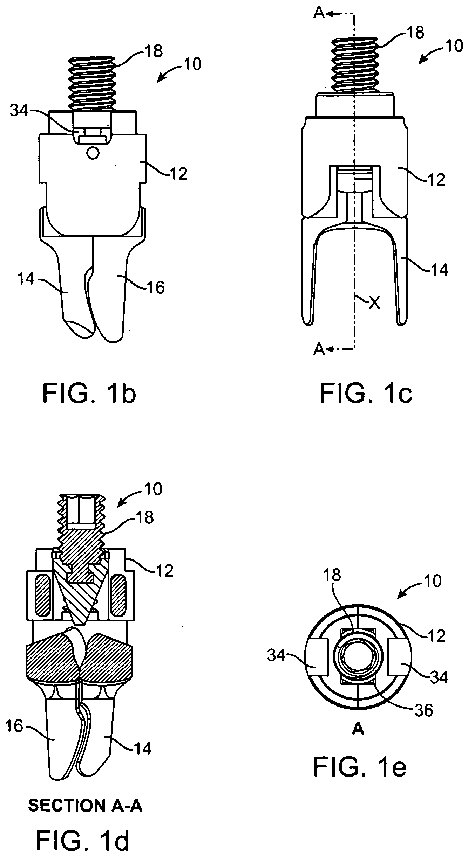

The outside of the body 12 defines a ledge 32 along at least a portion of the periphery. Notches 34 are formed at opposite locations as also shown in FIG. 1b. The notches 34 are configured for pronged attachment to a spacer delivery instrument. When joined together, the left and right body pieces 20, 22 define a proximal opening 36 (as seen in FIG. 1e) and a distal opening 38 (as seen in FIG. 1a) in the body 12. A longitudinal scallop (not shown) extending from the proximal end of the spacer to the distal end is formed in the outer surface of the body 12 to facilitate placement of the spacer 10 between and to conform to the anatomy of adjacent interspinous processes.

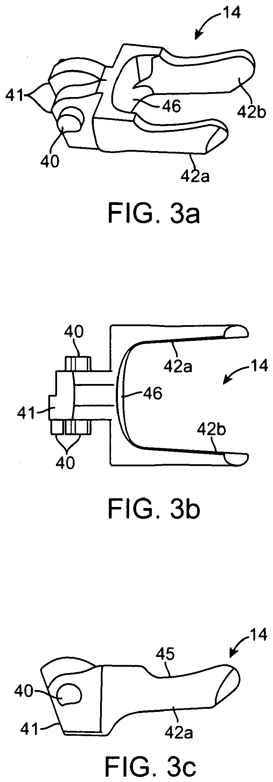

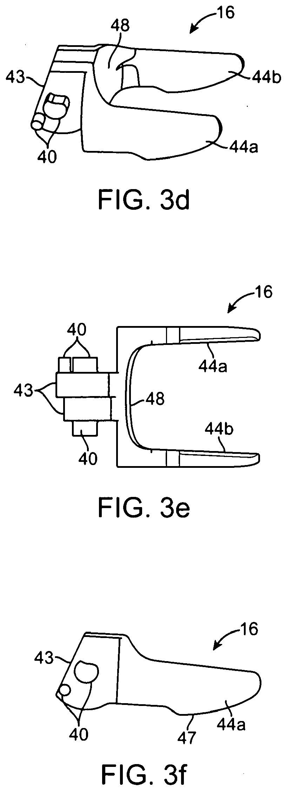

Turning now to FIGs. 3a-3c, the superior arm 14 is shown and in FIGs. 3d-3f, the inferior arm 16 is shown. The superior and inferior arms 14, 16 include pins 40 for mating with the body 12, in particular, for mating with the slots/openings 28 of the arm receiving portion 24. Each of the superior and inferior arms 14, 16 includes at least one caming surface 41, 43, respectively, for contact with the actuator assembly 18. The superior and inferior arms 14, 16 include elongated superior extensions 42a, 42b and elongated inferior extensions 44a, 44b, respectively. Extensions 42a and 44a are located on the left adjacent to the left body piece 20 and extensions 42b and 44b are located on right adjacent to the right body piece 22. Superior extensions 42a, 42b extend substantially parallel to each other in both an undeployed configuration and in a deployed configuration as do inferior extensions 44a, 44b. Extending between extensions 42a, 42b is a strut, bridge, bracket or saddle 46 that forms a superior substantially U-shaped configuration that is sized and configured to receive a superior spinous process. As seen in FIG. 3c, the anterior face of the superior extensions 14 includes a slight concavity or curvature 45 for conforming to the bony anatomy of the superior spinous process and or lamina. Also, extending between inferior extensions 44a, 44b is a strut, bridge, bracket or saddle 48 that forms an inferior substantially U-shaped configuration together with the extensions 44a, 44b that is sized and configured to receive an inferior spinous process of a spinal motion segment. As seen in FIG. 3f, the anterior face of the inferior extensions 16 includes a slight convexity or curvature 47 for conforming to the bony anatomy of the inferior spinous process and/or lamina.

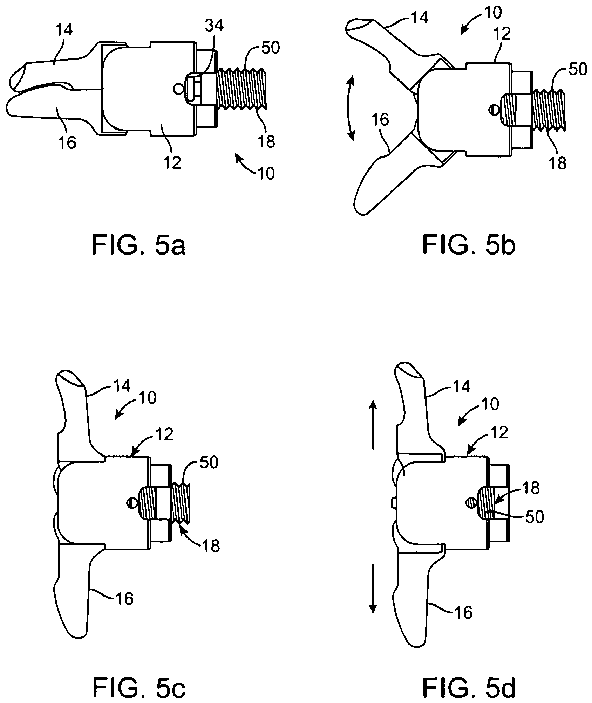

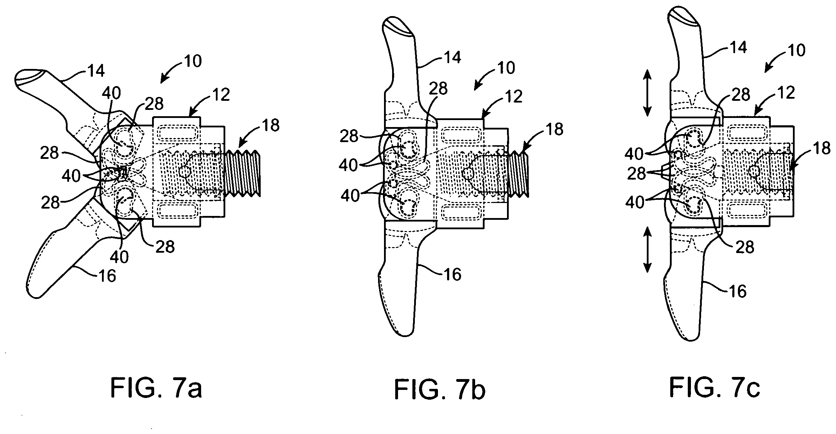

The superior and inferior arms 14, 16 are movably or rotatably connected to the body 12, for example by hinge means or the like to provide rotational movement from an undeployed configuration to a deployed configuration that arcs through about a 90 degree range or more with respect to the body 12. The arms 14, 16 are rotationally movable between at least an undeployed, collapsed or folded state (as shown in FIGs. 1a-1e) and at least one deployed state (as shown in FIGs. 5c, 5d, 6b, 6c, 7b, and 7c). In the undeployed state, the arm pairs 14, 16 are aligned generally or substantially axially (i.e., axially with the longitudinal axis, defined by the body 12 or to the translation path into the interspinous space of the patient) to provide a minimal lateral or radial profile. The longitudinal axis X of the spacer and body is shown in FIG. 1c. In the deployed state, the arm pairs 14, 16 are positioned such that each of the U-shaped saddles are in a plane (or planes) or have a U-shaped projection in a plane that is (are) generally or substantially transverse to the longitudinal axis X defined by the body 12 or to the collapsed position or to the implantation path into the interspinous space of the patient. In one variation, the spacer 10 is configured such the arms 14, 16 are linearly moveable or translatable within the plane from a first deployed state (such as the state shown in FIGs. 5c, 6b and 7b) to and from a second deployed state (such as the state shown in FIG. 5d, 6c and 7c) characterized by an additional translation of at least one of the arms 14, 16 with respect to the body 12 along a direction of the arrows as shown in FIG. 7c. More specifically, the arms 14, 16 can be extended in the general vertical direction along an axis along the general length the spine wherein the arms 14, 16 are extended away from each other and away from the body 12 as denoted by the arrows in FIG. 7c. This feature advantageously allows for the most minimally invasive configuration for the spacer without compromising the ability of the spacer 10 to seat and contain the spinous processes in between levels where the anatomy of the spinous processes is such that the interspinous process space increases in the anterior direction or without compromising the ability of the spacer to provide adequate distraction. The arms 14, 16 are connected to the body 12 and/or to each other in a manner that enables them to be moved simultaneously or independently of each other, as well as in a manner that provides passive deployment and/or vertical extension or, alternatively, active or actuated deployment and/or vertical extension.

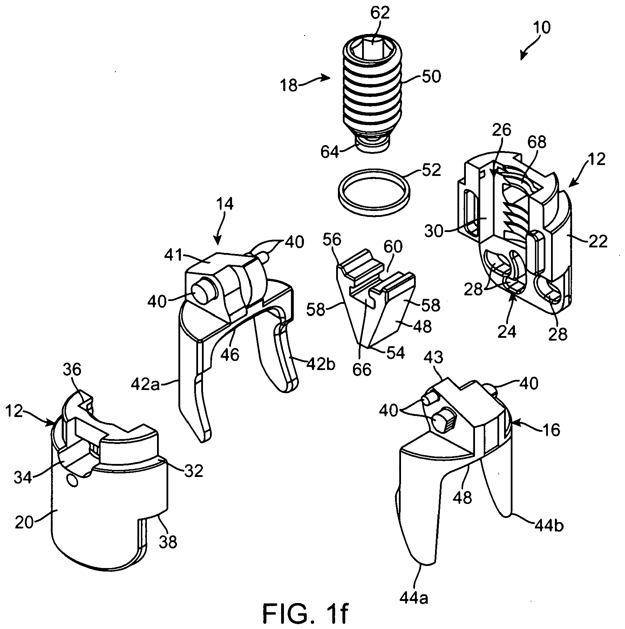

Turning back to FIG. 1f, the actuator assembly 18 will now be described. The actuator assembly 18 includes an actuator 48 connected to a shaft 50 and retainer 52. The actuator 48 includes a distal end 54 and a proximal end 56 and at least two bearing surfaces 58. The bearing surfaces 58 angle towards each other from the proximal end 54 to the distal end 56. The proximal end 54 of the actuator 48 includes a shaft receiving portion 60 configured to receive the shaft 50. The distal end of the actuator 48 is further configured to engage the superior and inferior arms 14, 16 such that forward translation of the actuator 48 relative to the body 12 effects deployment of the arms into at least one deployed configuration.

Still referencing FIG. 1f, the shaft 50 is substantially cylindrical in shape and includes a threaded outer surface for engagement with the threaded inner surface of the actuator assembly receiving portion 26 of the body 12. The threads on the inner surface of the body 12 are formed by the conjunction of both left and right body pieces 20, 22. The proximal end of the shaft 50 includes a hex socket 62 for receiving a driving tool for advancing the actuator assembly 18 with respect to the body 12. The distal end of the shaft 50 includes an actuator engagement portion 64 configured to connect to the actuator 48. The actuator engagement portion 64, as shown in FIG. 1f, is a projection that slides into a channel 66 on the actuator 48. Once inserted into the channel 66, movement of the shaft 50 solely along the longitudinal axis of the spacer 10 will not release the shaft 50 from the actuator 48. In one variation, the actuator 48 and shaft 50 are integrally formed.

Still referencing FIG. 1f, the retainer 52 is a circular ring preferably made of metal such as surgical steel or titanium. The retainer 52 fits into a recess 68 formed on the inner surface of the body 12. When pressed into the recess 68, the retainer 52 secures the actuator 48 inside the passageway 30 of the body 12. The actuator assembly 18 is at least partially disposed inside the body 12 and is configured for sliding engagement with respect to the body 12.

Assembly of the spacer 10 with reference to FIGs. 1a-1f will now be described. The arms 14, 16 are disposed in the arm receiving portion 24 of one body piece. The other of the left or right body piece 20, 22 is securely connected/welded to the one body piece thereby capturing the arms 14, 16 inside the arm receiving portion 24 such that the arms 14, 16 are capable of at least rotational movement with respect to the body 12 and in one variation, capable of rotational movement and translation with respect to the body 12. In a variation in which the body 12 is made of one piece, the arms 14, 16 are movably connected to the body 12 with a pin. The shaft 50 is connected to the actuator 48 and together inserted and threadingly connected into the passageway 30 of the body 12. The retainer 52 is passed over the proximal end of the shaft 50 and snapped into the recess 68 of the body 12 to secure the actuator assembly 18 inside the body 12 such that the actuator assembly 18 is capable of threaded translational movement with respect to the body 12.

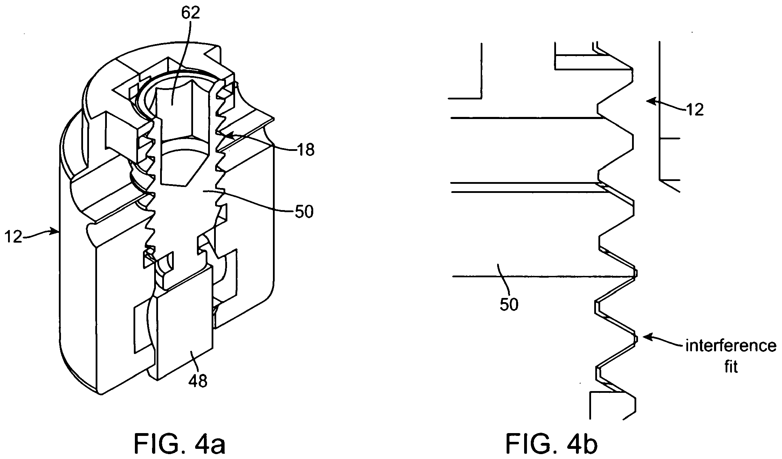

With particular reference to FIGs. 4a and 4b, in one variation, the threaded shaft 50 contacts the threaded inner surface of the body 12 in an interference fit engagement wherein the inner diameter of the body threads is smaller than the root diameter of the shaft threads as shown in FIG. 4b. This interference helps prevent the shaft 50 from loosening and backing-up out of the body 12 which would result in folding of the arms 14, 16. The interference is created with the threads of the shaft 50 and one or more of the distally located threads along the body 12 such that when advancement of the shaft 50 nears complete deployment (at approximately 90 degrees splaying of the arms 14, 16 with respect to the body 12) the distally located "interference" threads are engaged to tighten the shaft 50 to the body 12, thereby, providing an interference lock which is reversible by turning the shaft in the opposite direction. The interference lock with one or more of the distally located threads affords the surgeon flexibility in that the spacer 10 can be partially deployed and undeployed (via rotation of the shaft 50 in the opposite direction) repeatedly until the surgeon locates and properly seats the implant before locking it by advancing the shaft further into the section of the threads where the interference fit is created.

Referring now to FIGs. 5a-5d, the spacer 10 is shown in a closed, undeployed configuration (FIG. 5a), a partially deployed configuration or otherwise intermediary configuration (FIG. 5b), a deployed configuration (FIG. 5c), and a deployed and extended configuration (FIG. 5d). In moving from an undeployed to a deployed configuration, the actuator assembly 18, and in particular, the shaft 50 of the actuator assembly moves distally with respect to the body 12 and the shaft 50 advantageously moves to a position flush with the proximal end of the body 12 or to a position completely inside the body 12 disappearing from sight providing a low profile for the spacer 10 along the longitudinal axis of the body 12.

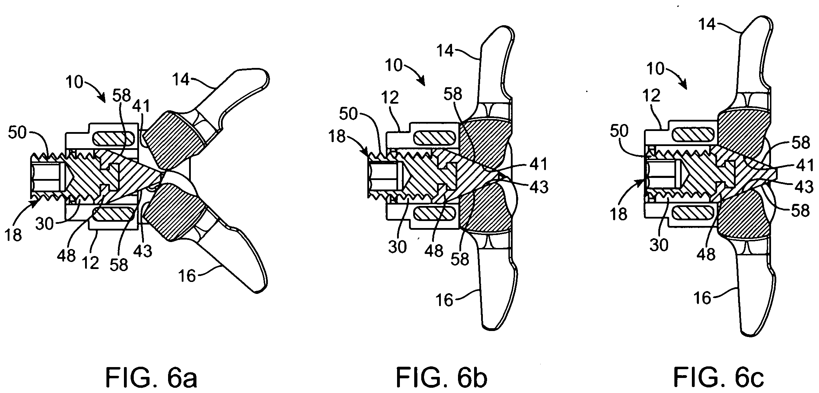

Turning now to the cross-sectional views of the spacer 10 in FIGs. 6a-6c, as the shaft 50 advances within the passageway 30, the bearing surfaces 58 of the actuator 48 contact the superior and inferior caming surfaces 41, 43 of the superior and inferior arms 14, 16 turning the arms 14, 16 into rotation with respect to the body 12. Upon rotation, the bearing surfaces 58 of the actuator 48 slide with respect to the superior and inferior caming surfaces 41, 43 of the superior and inferior arms 14, 16. The arms 14, 16 rotate through an arc of approximately 90 degrees with respect to the body 12 into the deployed configuration (FIG. 6b) and with further actuation, into a deployed and extended configuration (FIG. 6c) in which the superior and inferior extensions of the arms 14, 16 are substantially perpendicular to the longitudinal axis of the spacer 10 as shown in FIGs. 6b and 6c.

Turning now to the semi-transparent views of the spacer 10 in FIGs. 7a-7c, the rotation of the pins 40 of the arms 14, 16 in the slots 28 of the body 12 is shown in moving from the configuration of FIG. 7a to the configuration of FIG. 7b. The translation of the pins 40 of the arms 14, 16 in the elongated portion of the slots 28 of the body 12 is shown in moving from the deployed configuration of FIG. 7b to the deployed and extended configuration of FIG. 7c in the direction of the arrows in FIG. 7c. Such outward translation with respect to the body 12 is guided by the length and shape of the slots 28. Reverse rotation of the shaft 50 moves the shaft 50 proximally with respect to the body 12 allowing the arms to close to any intermediary configuration between a deployed, extended configuration and an undeployed, closed configuration. This feature advantageously permits the surgeon to ease installation and positioning of the spacer with respect to patient anatomy.

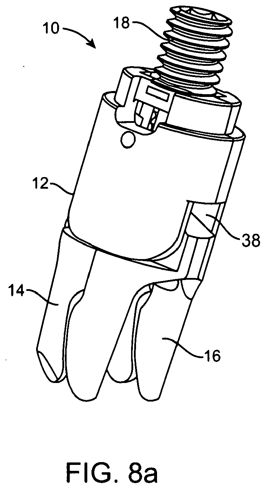

With reference to FIGs. 8a-8f, various views of another variation of a spacer 10 according to the present invention are shown wherein like reference numbers are used to describe like parts. The spacer 10 includes a body 12 connected to a superior extension member or arm 14, an inferior extension member or arm 16, and an actuator assembly 18.

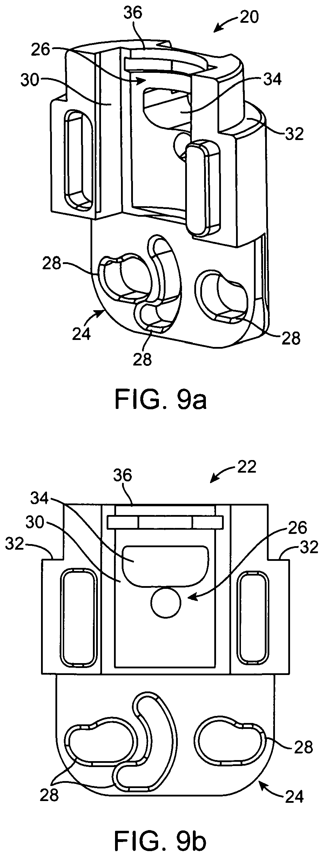

Turning now to FIGs. 9a and 9b, the body 12 will now be described. The body 12 is shown to have a clamshell construction with a left body piece 20 (shown in FIG. 9a) joined to a right body piece 22 (shown in FIG. 9b) to capture arms 14, 16 inside. With the right and left body pieces 20, 22 joined together, the body 12 is generally cylindrical. It has a cross-sectional size and shape that allows for implantation between adjacent spinous processes and facilitates delivery into a patient through a narrow port or cannula.

The inside of the body 12 defines an arm receiving portion 24 and an actuator assembly receiving portion 26 with features formed in each of the left and right body pieces 20, 22 that together define the arm and actuator assembly receiving portions 24, 26. In one variation, the arm receiving portion 24 includes slots or openings or apertures 28 that receive pins formed on the arms 14, 16 such that the pins rotate and/or translate inside the slots or apertures 28. The actuator assembly receiving portion 26 includes a passageway 30. Other features include a tongue and groove for mating with the opposite clamshell.

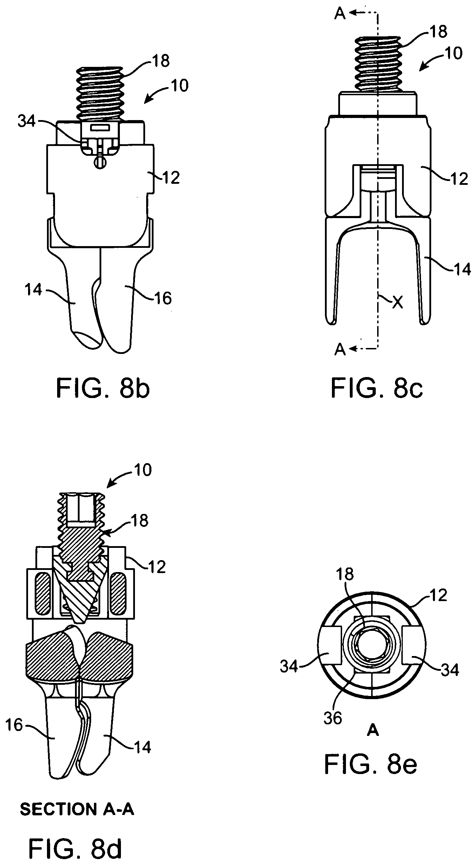

The outside of the body 12 defines a ledge 32 along at least a portion of the periphery. Notches 34 are formed at opposite locations as also shown in FIG. 8b. The notches 34 are configured for pronged attachment to a spacer delivery instrument. When joined together, the left and right body pieces 20, 22 define a proximal opening 36 (as seen in FIG. 8e) and a distal opening 38 (as seen in FIG. 8a) in the body 12. A longitudinal scallop (not shown) extending from the proximal end of the spacer to the distal end is formed in the outer surface of the body 12 to facilitate placement of the spacer 10 between and to conform to the anatomy of adjacent interspinous processes.

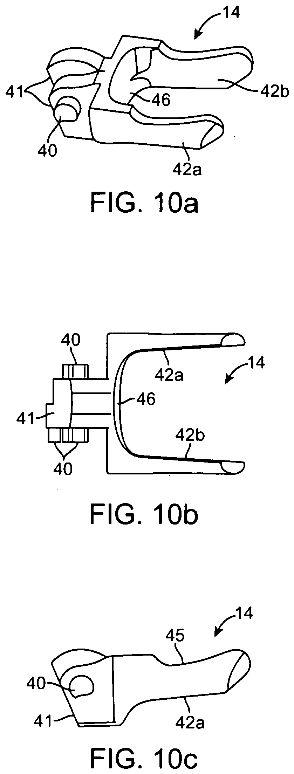

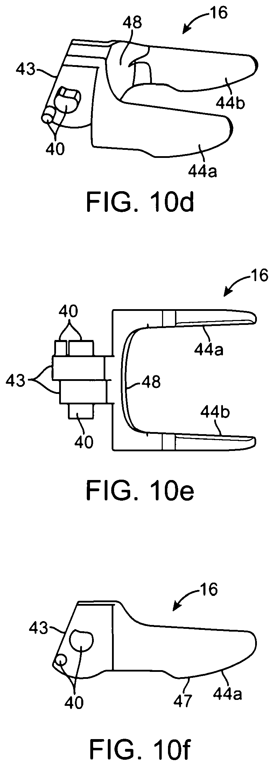

Turning now to FIGs. 10a-10c, the superior arm 14 is shown and in FIGs. 10d-10f, the inferior arm 16 is shown. The superior and inferior arms 14, 16, include pins 40 for mating with the body 12, in particular, for mating with the slots or apertures 28 of the arm receiving portion 24. Each of the superior and inferior arms 14, 16 includes at least one caming surface 41, 43, respectively, for contact with the actuator assembly 18. The superior and inferior arms 14, 16 include elongated superior extensions 42a, 42b and elongated inferior extensions 44a, 44b, respectively. Extensions 42a and 44a are located on the left adjacent to the left body piece 20 and extensions 42b and 44b are located on right adjacent to the right body piece 22. Superior extensions 42a, 42b extend substantially parallel to each other in both an undeployed configuration and in a deployed configuration as do inferior extensions 44a, 44b. Extending between extensions 42a, 42b is a strut, bridge, bracket or saddle 46 that forms a superior substantially U-shaped configuration that is sized and configured to receive a superior spinous process. As seen in FIG. 10c, the anterior face of the superior extensions 14 includes a slight concavity or curvature 45 for conforming to the bony anatomy of the superior spinous process and or lamina. Also, extending between inferior extensions 44a, 44b is a strut, bridge, bracket or saddle 48 that forms an inferior substantially U-shaped configuration together with the extensions 44a, 44b that is sized and configured to receive an inferior spinous process of a spinal motion segment. As seen in FIG. 10f, the anterior face of the inferior extensions 16 includes a slight convexity or curvature 47 for conforming to the bony anatomy of the inferior spinous process and or lamina.

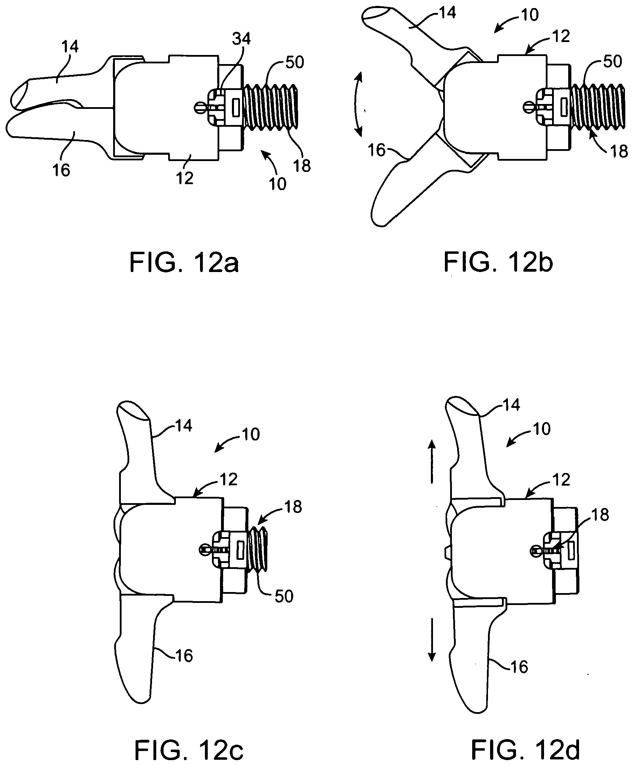

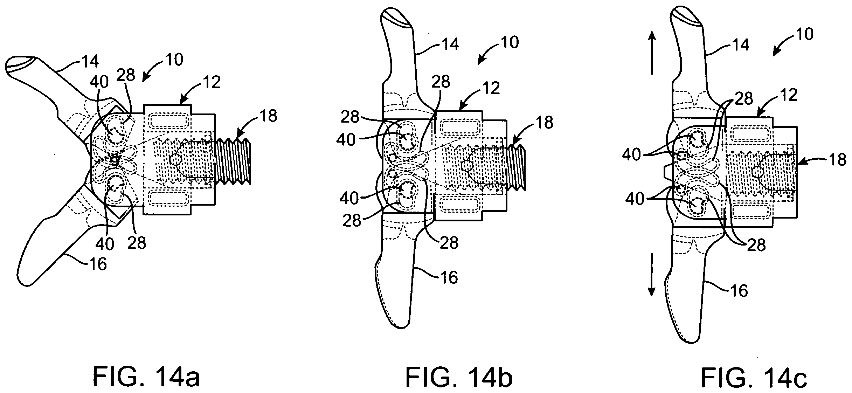

The superior and inferior arms 14, 16 are movably or rotatably connected to the body 12, for example by hinge means or the like to provide rotational movement from an undeployed configuration to a deployed configuration in which the arms arc through about a 90 degree range or more with respect to the body 12. The arms 14, 16 are rotationally movable between at least an undeployed, collapsed or folded state (as shown in FIGs. 8a-8e) and at least one deployed state (as shown in FIGs. 12c, 12d, 13b, 13c, 14b and 14c). In the undeployed state, the arm pairs 14, 16 are aligned generally or substantially axially (i.e., axially with the longitudinal axis defined by the body 12 or to the translation path into the interspinous space of the patient) to provide a minimal lateral or radial profile. The longitudinal axis X of the spacer and body is shown in FIG. 8c. In the deployed state, the arm pairs 14, 16 are positioned such that each of the U-shaped saddles are in a plane(s) or have a U-shaped projection in a plane that is generally or substantially transverse to the longitudinal axis X defined by the body 12 or to the collapsed position or to the implantation path into the interspinous space of the patient. In one variation, the spacer 10 is configured such the arms 14, 16 are linearly moveable or translatable within the plane from a first deployed state (such as the state shown in FIGs. 12c, 13b, 14b) to and from a second deployed state (such as the state shown in FIG. 12d, 13c, 14c) characterized by an additional translation of at least one of the arms 14, 16 with respect to the body 12 along a direction of the arrows as shown in FIG. 14c. More specifically, the arms 14, 16 can be extended in the general vertical direction along an axis substantially parallel to the spine wherein the arms 14, 16 are extended away from each other and away from the body 12 as denoted by the arrows in FIG. 14c. This feature advantageously allows for the most minimally invasive configuration for the spacer without compromising the ability of the spacer 10 to seat and contain the spinous processes in between levels where the anatomy of the spinous processes is such that the interspinous process space increases in the anterior direction or without compromising the ability of the spacer to provide adequate distraction. The arms 14, 16 are connected to the body 12 and/or to each other in a manner that enables them to be moved simultaneously or independently of each other, as well as in a manner that provides passive deployment and/or vertical extension or, alternatively, active or actuated deployment and/or vertical extension.

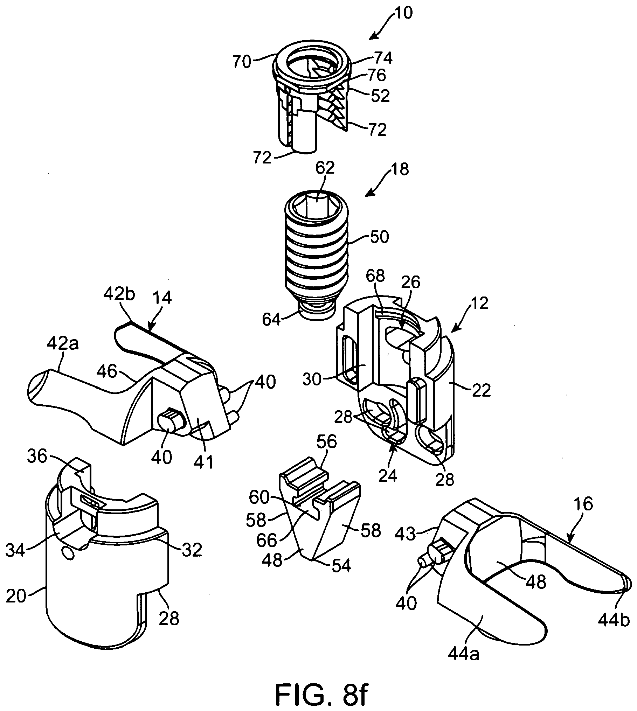

Turning back to FIG. 8f, the actuator assembly 18 will now be described. The actuator assembly 18 includes an actuator 48 connected to a shaft 50 and retainer 52. The actuator 48 includes a distal end 54 and a proximal end 56 and at least two bearing surfaces 58. The bearing surfaces 58 angle towards each other from the proximal end 54 towards the distal end 56. The proximal end 54 of the actuator 48 includes a shaft receiving portion 60 configured to receive the shaft 50. The distal end of the actuator 48 is further configured to engage the superior and inferior arms 14, 16 such that forward translation of the actuator 48 relative to the body 12 effects deployment of the arms into at least one deployed configuration.

Still referencing FIG. 8f, the shaft 50 is substantially cylindrical in shape and includes a threaded outer surface for engagement with a threaded inner surface of the retainer 52. The proximal end of the shaft 50 includes a hex socket 62 for receiving a driving tool for advancing the actuator assembly 18 with respect to the body 12 to deploy the arms 14, 16. The distal end of the shaft 50 includes an actuator engagement portion 64 configured to connect to the actuator 48. The actuator engagement portion 64 as shown in FIG. 8f, is a projection that slides into a channel 66 on the actuator 48. Once inserted into the channel 66, movement of the shaft 50 solely along the longitudinal axis of the spacer 10 will not release the shaft 50 from the actuator 48. In one variation, the actuator 58 and shaft 50 are integrally formed.

Still referencing FIG. 8f, the retainer 52, which is preferably made of metal such as surgical steel or titanium, includes a circular proximal end 70 and two prongs 72 extending distally from the circular proximal end 70 configured to received the shaft 50. Clearance for the passage of the actuator 48 is provided between the prongs 72. Threads are formed on the inner surface of the prongs 72 for conformal engagement with the threads on the shaft 50 such that the shaft 50 may threadingly pass through the retainer 52. In one variation each prong 72 is split down the middle as shown in FIG. 8f. A ledge 74 is formed along at least a portion of the periphery of the retainer 52 at the proximal end. The ledge 74 of the retainer 52 fits into a recess 68 formed on the inner surface of the body 12. When pressed into the recess 68, the retainer 52 is secured to the body 12 and the retainer 52 in turn secures the actuator 48 inside the passageway 30 of the body 12. Alignment flats 76 are formed at locations opposite to each other on the outer surface of the retainer 52. The alignment flats 76 keep the threaded insert aligned with the body 12.

Assembly of the spacer 10 with reference to FIG. 8f will now be described. The arms 14, 16 are disposed in the arm receiving portion 24 of one body piece. The other of the left or right body piece 20, 22 is securely connected/welded to the one body piece thereby capturing the arms 14, 16 inside the arm receiving portion 24 such that the arms 14, 16 are capable of rotational movement with respect to the body 12 and, in one variation, capable of rotational movement and translation with respect to the body 12. The shaft 50 threaded into the retainer 52 and connected to the actuator 58. The retainer 52 and the actuator 48 and shaft 50 are inserted into the passageway 30 of the body 12 and the retainer 52 is snapped inside the recess 68 of the body 12 to secure the actuator 48 inside the body 12 such that the actuator assembly 18 is capable of threaded translational movement with respect to the body 12.

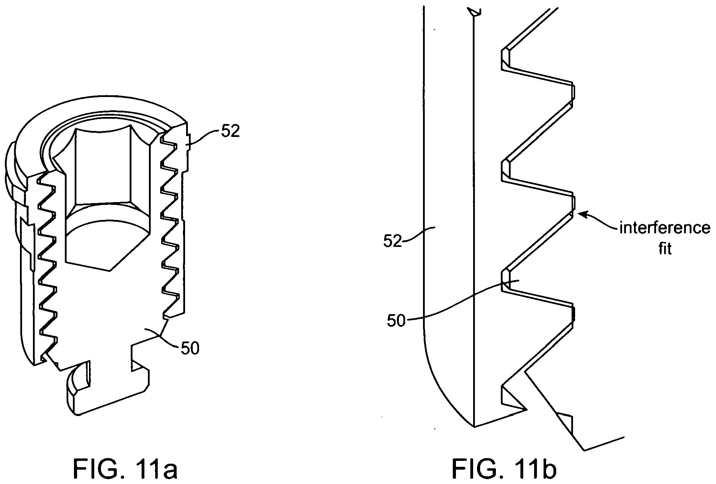

With particular reference to FIGs. 11a and 11b, the threaded shaft 50 contacts the threaded inner surface of the retainer 52 in an interference fit engagement wherein the inner diameter of the retainer threads is smaller than the root diameter of the shaft threads as shown in FIG. 11b. This interference helps prevent the shaft 50 from loosening and backing-up out of the body 12 which would result in folding of the arms 14, 16. The interference is created with the threads of the shaft 50 and one or more of the threads along the retainer 52 such that when advancement of the shaft 50 nears complete deployment (at approximately 90 degrees splaying of the arms 14, 16 with respect to the body 12) the distally located "interference" threads relative to the more proximally located non-interference threads of the same retainer 52 are engaged to tighten the shaft 50 to the retainer 52 and in turn to the body 12, thereby, providing an interference lock which is reversible by turning the shaft in the opposite direction. The prongs 72 of the retainer 52 advantageously and reversibly flex outwardly and inwardly instead of plastically deforming. This feature allows the implant to be repeatedly locked and unlocked without wearing out the threads and thereby preserving the interference lock feature. The interference lock with one or more of the distally located threads affords the surgeon flexibility in that the spacer 10 can be partially deployed and undeployed (via rotation of the shaft 50 in the opposite direction) repeatedly until the surgeon locates and properly seats the implant before locking it by advancing the shaft further into the section of the threads where the interference fit is created.

Referring now to FIGs. 12a-12d, the spacer 10 is shown in a closed, undeployed configuration (FIG. 12a), a partially deployed configuration, or intermediary configuration (FIG. 12b), a deployed configuration (FIG. 12c), and a deployed and extended configuration (FIG. 12d). In moving from an undeployed to a deployed configuration, the actuator assembly 18, and in particular, the shaft 50 of the actuator assembly moves distally with respect to the body 12 and the shaft 50 advantageously moves to a position flush with the proximal end of the body 12 or to a position completely inside the body 12 disappearing from sight providing a low profile for the spacer 10 along the longitudinal axis of the body 12.

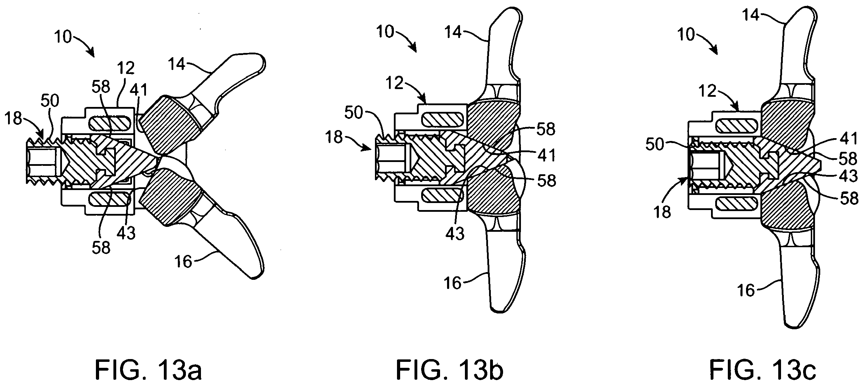

Turning now to the cross-sectional views of the spacer 10 in FIGs. 13a-13c, as the shaft 50 advances within the passageway 30, the bearing surfaces 58 of the actuator 48 contact the superior and inferior caming surfaces 41, 43 of the superior and inferior arms 14, 16 turning the arms 14, 16 into rotation with respect to the body 12. Upon rotation, the bearing surfaces 58 of the actuator 48 slide with respect to the superior and inferior caming surfaces 41, 43 of the superior and inferior arms 14, 16. The arms 14, 16 rotate through an arc of approximately 90 degrees with respect to the body 12 into the deployed configuration (FIG. 13b) and with further actuation, into a deployed and extended configuration (FIG. 13c) in which the superior and inferior extensions of the arms 14, 16 are substantially perpendicular to the longitudinal axis of the spacer 10 as shown in FIGs. 13b and 13c.

Turning now to the semi-transparent views of the spacer 10 in FIGs. 14a-14c, the rotation of the pins 40 of the arms 14, 16 in the slots 28 of the body 12 is shown in moving from the configuration of FIG. 14a to the configuration of FIG. 14b. The translation of the pins 40 of the arms 14, 16 in the elongated portion of the slots 28 of the body 12 is shown in moving from the deployed configuration of FIG. 14b to the deployed and extended configuration of FIG. 14c in the direction of the arrows in FIG. 14c. Such outward translation with respect to the body 12 is guided by the length and shape of the slots 28. Reverse rotation of the shaft 50 moves the shaft 50 proximally with respect to the body 12 allowing the arms to close to any intermediary configuration between a deployed, extended configuration and an undeployed, closed configuration. This feature advantageously permits the surgeon to ease installation and positioning of the spacer with respect to patient anatomy.

The spacer of FIGs. 8-14 is delivered and deployed in the same manner as the spacer of FIGs. 1-7. To deliver and deploy the spacer 10 within the patient, the spacer 10 is releasably attached to an insertion instrument 80 at the proximal end of the spacer 10 via notches 34. The insertion instrument 80 includes a first assembly 102 connected to a second assembly 104 and a handle assembly 106.

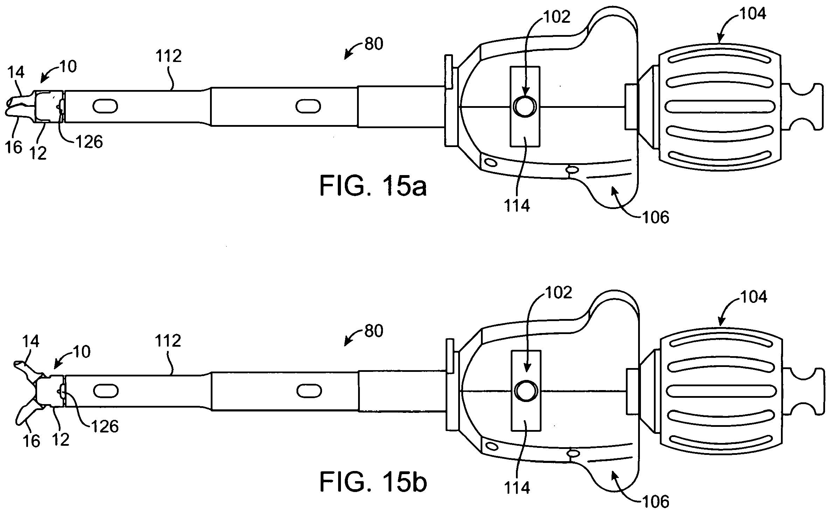

The spacer 10 is provided or otherwise placed in its undeployed, closed state in juxtaposition to the insertion instrument 80 and connected thereto as shown in FIG. 15a. The longitudinal axis of the insertion instrument 80 is advantageously substantially aligned with the longitudinal axis of the spacer 10 as shown. The delivery instrument 80 includes a first subassembly 102 to releasably clamp to the body 12 of the spacer 10 at a distal end of the insertion instrument 80. The first subassembly 102 includes an inner clamp shaft (not shown) having flexible prongs 126 at the distal end configured for attachment to the body 12 of the spacer 10 and, in particular, for insertion into the notches 34 of the spacer body 12. The first subassembly 102 includes an outer shaft 112 located over the inner clamp shaft and configured for relative motion with respect to one another via a control 114 located at the handle assembly 106. The control 114 is threaded to the outer shaft 112 such that rotation of the control 114 moves the outer shaft 112 along the longitudinal axis of the insertion instrument 80 over the inner clamp shaft to deflect and undeflect the prongs 126 to connect or disconnect the instrument 80 to or from the body 12. The first control 114 is activated at the handle of the insertion instrument 100 such that the first subassembly 102 is connected to the body 12 of the spacer 10. The first control 114 is rotated in one direction to advance the outer shaft 112 over the inner clamp shaft 110 deflecting the prongs 118 inwardly into the notches 34 on the body of the spacer 12 to secure the spacer body 12 to the instrument as shown clearly in FIG. 15a. Reverse rotation of the control 114 reverses the direction of translation of the outer shaft 112 to release the prongs 126 from the notches 34 and, thereby, release the spacer 10 from the instrument 80.

Still referencing FIG. 15a, the insertion instrument 80 includes a second subassembly 104 that is configured to connect to the actuator assembly 18 of the spacer 12. In particular, the second subassembly 104 includes means located at the distal end of the second subassembly 104 to activate the actuator assembly 18. In one variation, the second subassembly 104 is a hexagonal-shaped driver having an elongated shaft that is configured to be insertable into the hex socket 62 of the shaft 50 while the spacer 10 is connected to the instrument 80. The second subassembly 104 is insertable at the proximal end of the instrument 80 and extends through the handle assembly 106 and through the inner shaft. The removable driver 104 is rotatable with respect to the instrument 80 to arrange the spacer 10 to and from deployed and undeployed configurations.

To deliver and deploy the spacer 10 within the patient, the spacer 10 is releasably attached to a delivery instrument 80 at the proximal end of the spacer 10 as described. A small midline or lateral-to-midline incision is made in the patient for minimally-invasive percutaneous delivery. In one variation, the supraspinous ligament is split longitudinally along the direction of the tissue fibers to create an opening for the instrument. Dilators may be further employed to create the opening. In the undeployed state with the arms 14, 16 in a closed orientation and attached to a delivery instrument, the spacer 10 is inserted into a port or cannula, if one is employed, which has been operatively positioned to an interspinous space within a patient's back and the spacer is passed through the cannula to the interspinous space between two adjacent vertebral bodies. The spacer 10 is advanced beyond the end of the cannula or, alternatively, the cannula is pulled proximately to uncover the spacer 10 connected to the instrument 80. Once in position, the second assembly 104 is inserted into the instrument 80 and is rotated to begin the deployment of at least one of the superior arm 14 and inferior arm 16 or both simultaneously. FIG. 15b illustrates the superior arm 14 and the inferior arm 16 in a partially deployed position with the arms 14, 16 rotated away from the longitudinal axis. Rotation of the driver 104 turns the actuator shaft 50 advancing it with respect to the body 12 which distally advances the actuator 48 whose bearing surfaces 58 contact the superior and inferior camming surfaces 41, 43 pushing the superior and inferior arms 14, 16 into rotation about the pins 40. The position of the arms 14, 16 in FIG. 15b may be considered to be one of many partially deployed configurations or intermediary configurations that are possible and from which the deployment of the arms 14, 16 is reversible with opposite rotation of the second assembly 104.

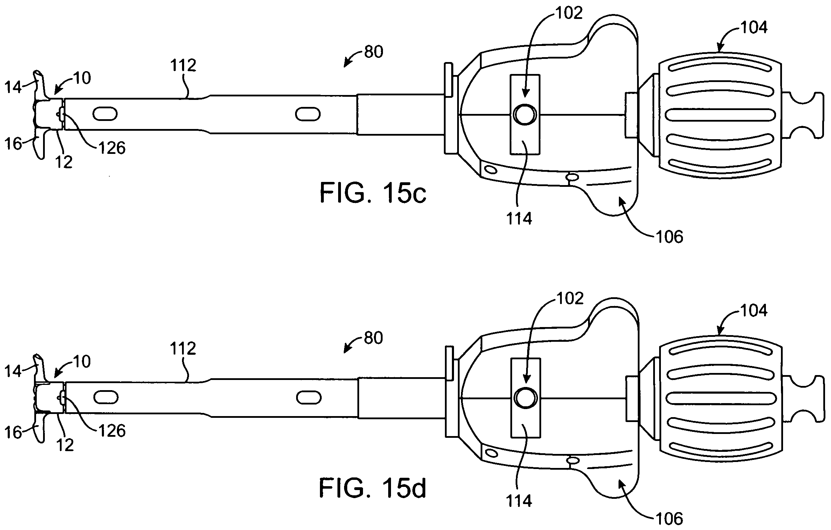

Turning to FIG. 15c, there is shown an insertion instrument 80 connected to a spacer 10 in a first deployed configuration in which the arms 14, 16 are approximately 90 degrees perpendicular to the longitudinal axis or perpendicular the initial undeployed configuration. Continued rotation of second assembly 104 threads the shaft 50 further distally with respect to the body 12 of the spacer 10 pushing the bearing surfaces 58 further against the superior and inferior camming surfaces 41, 43. While in the first deployed configuration, the clinician can observe with fluoroscopy the positioning of the spacer 10 inside the patient and then choose to reposition the spacer 10 if desired. Repositioning of the spacer may involve undeploying the arms 14, 16 rotating them into any one of the many undeployed configurations. The spacer may then be re-deployed into the desired location. This process can be repeated as necessary until the clinician has achieved the desired positioning of the spacer in the patient.

Even further advancement of the actuator shaft 50 via rotation of the second subassembly 104 from the first deployed configuration results in the spacer 10 assuming a second deployed configuration shown in FIG. 15d. The second deployed configuration is an extended configuration in which the superior and inferior arms 14, 16 extend transversely with respect to the longitudinal axis outwardly in the direction of the arrows in FIG. 14c. Such extension is guided by the length and shape of the slots 28 in which the arms 14, 16 move. Once deployed, the superior arm 14 seats the superior spinous process and the inferior arm 16 seats the adjacent inferior spinous process. Such extension may also provide some distraction of the vertebral bodies.

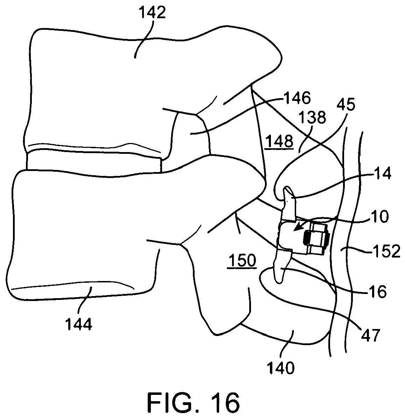

Following deployment, the second assembly 104 may be removed. Control 114 is rotated in the opposite direction to release the body 12 from the instrument 80. The insertion instrument 80, thus released from the spacer 10, is removed from the patient leaving the spacer 10 implanted in the interspinous process space as shown in FIG. 16. In FIG. 16, the spacer 10 is shown with the superior arm 14 seating the superior spinous process 138 of a first vertebral body 142 and the inferior arm 16 seating the inferior spinous process 140 of an adjacent second vertebral body 144 providing sufficient distraction to open the neural foramen 146 to relieve pain. As mentioned above, the shape of the superior arm 14 is such that a superior concavity or curvature 45 is provided to conform to the widening of the superior spinous process 138 in an anterior direction toward the superior lamina 148 going in the anterior direction. In general, the superior arm 14 is shaped to conform to anatomy in the location in which it is seated. Likewise, as mentioned above, the shape of the inferior arm 16 is such that an inferior convexity or curvature 47 is provided to conform to the widening of the inferior spinous process 140 in an anterior direction toward the inferior lamina 150. The supraspinous ligament 152 is also shown in FIG. 20

The spacer 10 is as easily and quickly removed from body of the patient as it is installed. The instrument 80 is inserted into an incision and reconnected to the spacer 10. The shaft 50 is rotated in the opposite direction via a driver 104 to fold the arms 14, 16 into a closed or undeployed configuration. In the undeployed configuration, the spacer 10 can be removed form the patient along with the instrument 80 or, of course, re-adjusted and re-positioned and then re-deployed as needed with the benefit of minimal invasiveness to the patient.

Any of the spacers disclosed herein are configured for implantation employing minimally invasive techniques including through a small percutaneous incision and through the superspinous ligament. Implantation through the superspinous ligament involves selective dissection of the superspinous ligament in which the fibers of the ligament are separated or spread apart from each other in a manner to maintain as much of the ligament intact as possible. This approach avoids crosswise dissection or cutting of the ligament and thereby reduces the healing time and minimizes the amount of instability to the affected spinal segment. While this approach is ideally suited to be performed through a posterior or midline incision, the approach may also be performed through one or more incisions made laterally of the spine with or without affect to the superspinous ligament. Of course, the spacer may also be implanted in a lateral approach that circumvents the superspinous ligament altogether as well as in open or mini-open procedures.

For example, a spacer may include only a single arm which is configured to receive either the superior spinous process or the inferior spinous process. The surface of the spacer body opposite the side of the single arm may be contoured or otherwise configured to engage the opposing spinous process wherein the spacer is sized to be securely positioned in the interspinous space and provide the desired distraction of the spinous processes defining such space. The additional extension of the arm(s) subsequent to their initial deployment in order to seat or to effect the desired distraction between the vertebrae may be accomplished by expanding the body portion of the device instead of or in addition to extending the individual extension members 14, 16.

The extension arms of the subject device may be configured to be selectively movable subsequent to implantation, either to a fixed position prior to closure of the access site or otherwise enabled or allowed to move in response to normal spinal motion exerted on the device after deployment. The deployment angles of the extension arms may range from less than 90 degrees (relative to the longitudinal axis defined by the device body) or may extend beyond 90 degrees and remain stationary or be dynamic. Each extension member may be rotationally movable within a range that is different from that of the other extension members. Additionally, the individual superior and/or inferior extensions 42a, 42b, 44a, 44b may be movable in any direction relative to the strut or bridge extending between an arm pair or relative to the device body in order to provide shock absorption and/or function as a motion limiter, or serve as a lateral adjustment particularly during lateral bending and axial rotation of the spine. The manner of attachment or affixation of the extensions to the arms may be selected so as to provide movement of the extensions that is passive or active or both. In one variation, the saddle or distance between extensions 42a and 42b or between 44a and 44b can be made wider to assist in seating the spinous process and then narrowed to secure the spinous process positioned between extensions 42a and 42b or between 44a and 44b.

Furthermore, all examples and conditional language recited herein are principally intended to aid the reader in understanding the principles of the invention and the concepts contributed by the inventors to furthering the art, and are to be construed as being without limitation to such specifically recited examples and conditions. Moreover, all statements herein reciting principles, aspects, and embodiments of the invention as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. The scope of the present invention, therefore, is not intended to be limited to the exemplary embodiments shown and described herein. Rather, the scope of present invention is embodied by the appended claims.

## Claims

1. An implantable spacer (10) for placement between adjacent spinous processes comprising:
a body (12) defining a longitudinal axis and passageway through at least a portion of the body (12);
a first arm (14) and a second arm (16) both connected to the body (12) and capable of rotation with respect to the body (12) such that the first and second arms (14,16) are rotatable away from each other and away from the longitudinal axis of the body (12);
each arm defining a substantially U-shaped configuration for receiving a spinous process;
each arm (14, 16) a proximal caming surface (41, 43);
an actuator assembly (18) connected to the body (12);
the actuator assembly (18) comprising:
an actuator (48) having at least one bearing surface (58); and
a threaded shaft (50) connected to the actuator (48) and configured for movement with respect to the body (12);
the actuator assembly (18) is configured such that the actuator (48) is disposed inside the body (12) and configured to move relative to the body (12) such that the at least one bearing surface contacts the caming surfaces (41, 43) of the arms (14, 16) to thereby rotate the arms (14, 16) from an undeployed configuration in which the arms (14, 16) are substantially parallel to the longitudinal axis of the body (12) to a deployed configuration in which the arms (14, 16) are substantially perpendicular to the longitudinal axis of the body (12);
wherein the arms (14, 16) are configured to receive and seat the adjacent spinous processes when in the deployed configuration.

2. The spacer of claim 1 further including a retainer (52) configured to retain the actuator (48) inside the body (12), wherein the retainer includes an inner threaded surface (60) configured for threaded engagement with the threaded shaft (50).

3. The spacer of claim 2 wherein the retainer includes two prongs with (72) inner threaded surfaces configured to receive the threaded shaft (50).

4. The spacer of claim 3 wherein at least one prong (72) is split.

5. The spacer of claims 3 and 4 wherein a clearance is defined between each prong (72) such that the actuator (48) is passable through the clearance.

6. The spacer of claims 3-5 wherein the two prongs (72) are connected at their proximal ends to a circular proximal end (70).

7. The spacer of claims 3-6 wherein the two prongs (72) are flexible with respect to the circular proximal end (70).

8. The spacer of claims 6 and 7 wherein the circular proximal end (70) is configured for attachment to the body (12) such that the actuator (48) and threaded shaft (50) is movably connected with respect to the body (12).

9. The spacer of claims 1-8 wherein the first and second arms (14, 16) are configured for rotation and translation with respect to the body (12).

10. The spacer of claims 1-9 wherein the apertures (28) are configured such that the first and second arms (14, 16) are capable of outwardly extending away from the body.

11. The spacer of claims 1-10 wherein the first and second arms (14, 16) rotate approximately 90 degrees into at least one deployed configuration in which the arms (14, 16) are approximately transverse to the longitudinal axis of the body (12);
said rotation being from an undeployed configuration in which the arms (14, 16) are substantially parallel to the longitudinal axis of the body (12).

12. The spacer of claims 1-11 wherein the first and second arms (14, 16) rotate approximately 90 degrees into at least one deployed configuration in which the arms (14, 16) are approximately transverse to the longitudinal axis of the body (12);
said rotation being from an undeployed configuration in which the arms (14, 16) are substantially parallel to the longitudinal axis of the body (12); and
wherein following said rotation upon further actuation the arms (14, 16) translate with respect to the body (12) in a direction substantially transverse to longitudinal axis of the body (12).

13. The spacer of claims 1-12 wherein the passageway includes an inner threaded surface configured for threaded engagement with the threaded shaft (50).

14. The spacer of claims 1-13 wherein the body (12) includes notches (34) configured for attachment to a spacer delivery instrument such that the longitudinal axis of the spacer delivery instrument is substantially aligned with the longitudinal axis of the spacer (10).

15. The spacer of claims 1-14 wherein rotation of the threaded shaft (50) in one direction advances the actuator(48) distally to engage the caming surfaces (41, 43) and deploy the arms (14, 16) into at least one deployed configuration and rotation of the threaded shaft (50) in an opposite direction moves the actuator (48) proximally to permit the arms (14, 16) to fold into at least one undeployed or intermediary configuration.

16. The spacer of claims 1-15 wherein each arm (14, 16) has a pair of extensions (42a, 42b, 44a, 44b) and a saddle (46) defining the substantially U-shaped configuration for seating a spinous process.

## Patentansprüche

1. Implantierbares Abstandsglied (10) zur Platzierung zwischen benachbarten spinösen Fortsätzen, umfassend:
einen Körper (12), der eine Längsachse und einen Durchgang durch mindestens einen Teil des Körpers (12) definiert;
einen ersten Arm (14) und einen zweiten Arm (16), die beide mit dem Körper (12) verbunden und zur Drehung bezogen auf den Körper (12) fähig sind, sodass der erste und zweite Arm (14, 16) voneinander weg und von der Längsachse des Körpers (12) weg drehbar sind;
wobei jeder Arm eine im Wesentlichen U-förmige Konfiguration zum Aufnehmen eines spinösen Fortsatzes definiert;
wobei jeder Arm (14, 16) eine proximale Nockenoberfläche (41, 43) aufweist;
eine Aktorbaugruppe (18), die mit dem Körper (12) verbunden ist;
wobei die Aktorbaugruppe (18) umfasst:
einen Aktor (48) mit mindestens einer Lageroberfläche (58); und
eine Gewindewelle (50), die mit dem Aktor (48) verbunden und für Bewegung bezogen auf den Körper (12) konfiguriert ist;
wobei die Aktorbaugruppe (18) so konfiguriert ist, dass der Aktor (48) innen im Körper (12) angeordnet und dafür konfiguriert ist, sich relativ zum Körper (12) zu bewegen, so dass die mindestens eine Lageroberfläche die Nockenoberflächen (41, 43) der Arme (14, 16) berührt, um dadurch die Arme (14, 16) von einer nicht eingesetzten Konfiguration, in der die Arme (14, 16) im Wesentlichen parallel zur Längsachse des Körpers (12) sind, in eine eingesetzte Konfiguration, in der die Arme (14, 16) im Wesentlichen senkrecht zur Längsachse des Körpers (12) sind, zu drehen;
worin die Arme (14, 16) dafür konfiguriert sind, die benachbarten spinösen Fortsätze, wenn in der eingesetzten Konfiguration, aufzunehmen und zu lagern.

2. Abstandsglied nach Anspruch 1, ferner beinhaltend einen Halter (52), der dafür konfiguriert ist, den Aktor (48) innen im Körper (12) zu halten, worin der Halter eine innere Gewindeoberfläche (60) beinhaltet, die für einen Gewindeeingriff in die Gewindewelle (50) konfiguriert ist.

3. Abstandsglied nach Anspruch 2, worin der Halter zwei Zacken (72) mit inneren Gewindeoberflächen beinhaltet, die dafür konfiguriert sind, die Gewindewelle (50) aufzunehmen.

4. Abstandsglied nach Anspruch 3, worin mindestens ein Zacken (72) geteilt ist.

5. Abstandsglied nach Anspruch 3 und 4, worin ein Freiraum zwischen jedem Zacken (72) definiert ist, sodass der Aktor (48) durch den Freiraum führbar ist.

6. Abstandsglied nach Anspruch 3-5, worin die beiden Zacken (72) an ihren proximalen Enden mit einem kreisförmigen proximalen Ende (70) verbunden sind.

7. Abstandsglied nach Anspruch 3-6, worin die beiden Zacken (72) bezogen auf das kreisförmige proximale Ende (70) flexibel sind.

8. Abstandsglied nach Anspruch 6 und 7, worin das kreisförmige proximale Ende (70) für die Anbringung am Körper (12) konfiguriert ist, sodass der Aktor (48) und die Gewindewelle (50) bezogen auf den Körper (12) bewegbar verbunden sind.

9. Abstandsglied nach Anspruch 1-8, worin der erste und zweite Arm (14, 16) für Drehung und Verschiebung bezogen auf den Körper (12) konfiguriert sind.

10. Abstandsglied nach Anspruch 1-9, worin die Aperturen (28) konfiguriert sind, sodass der erste und zweite Arm (14, 16) zum Erstrecken nach außen weg vom Körper fähig sind.

11. Abstandsglied nach Anspruch 1-10, worin sich der erste und zweite Arm (14, 16) ungefähr 90 Grad in mindestens eine eingesetzte Konfiguration drehen, in der die Arme (14, 16) ungefähr transversal zur Längsachse des Körpers (12) sind;
wobei die Drehung aus einer nicht eingesetzten Konfiguration erfolgt, in der die Arme (14, 16) im Wesentlichen parallel zur Längsachse des Körpers (12) sind.

12. Abstandsglied nach Anspruch 1-11, worin sich der erste und zweite Arm (14, 16) ungefähr 90 Grad in mindestens eine eingesetzte Konfiguration drehen, in der die Arme (14, 16) ungefähr transversal zur Längsachse des Körpers (12) sind;
wobei die Drehung aus einer nicht eingesetzten Konfiguration erfolgt, in der die Arme (14, 16) im Wesentlichen parallel zur Längsachse des Körpers (12) sind; und
worin nach der Drehung, bei weiterer Betätigung, sich die Arme (14, 16) bezogen auf den Körper (12) in einer Richtung verschieben, die im Wesentlichen transversal zur Längsachse des Körpers (12) ist.

13. Abstandsglied nach Anspruch 1-12, worin der Durchgang eine innere Gewindeoberfläche beinhaltet, die für einen Gewindeeingriff in die Gewindewelle (50) konfiguriert ist.

14. Abstandsglied nach Anspruch 1-13, worin der Körper (12) Kerben (34) beinhaltet, die für die Anbringung an einem Abstandsglied-Zuführungsinstrument konfiguriert sind, sodass die Längsachse des Abstandsglied-Zuführungsinstruments im Wesentlichen mit der Längsachse des Abstandsglieds (10) ausgerichtet ist.

15. Abstandsglied nach Anspruch 1-14, worin Drehung der Gewindewelle (50) in einer Richtung den Aktor (48) distal vorwärtsbewegt, um die Nockenoberflächen (41, 43) in Eingriff zu nehmen und die Arme (14, 16) in mindestens einer eingesetzter Konfiguration einzusetzen und Drehung der Gewindewelle (50) in einer entgegengesetzten Richtung den Aktor (48) proximal bewegt, damit sich die Arme (14, 16) in mindestens einer nicht eingesetzten oder intermediären Konfiguration zusammenklappen können.

16. Abstandsglied nach Anspruch 1-15, worin jeder Arm (14, 16) ein Paar Verlängerungen (42a, 42b, 44a, 44b) und einen Sattel (46), der die im Wesentlichen U-förmige Konfiguration zum Lagern eines spinösen Fortsatzes definiert, aufweist.

## Revendications

1. Écarteur implantable (10) destiné à être mis en place entre des processus épineux adjacents, comprenant :
un corps (12) définissant un axe longitudinal et un passage à travers au moins une partie du corps (12) ;
un premier bras (14) et un second bras (16) connectés l'un et l'autre au corps (12) et capables de pivoter par rapport au corps (12) de sorte que les premier et second bras (14,16) puissent pivoter à l'opposé l'un de l'autre par rapport à l'axe longitudinal du corps (12),
chaque bras définissant une configuration sensiblement en forme de U pour recevoir un processus épineux,
chaque bras (14, 16) ayant une surface de came proximale (41, 43) ;
un ensemble actionneur (18) connecté au corps (12),
l'ensemble actionneur (18) comprenant :
un actionneur (48) ayant au moins une surface d'appui (58) ; et
un arbre fileté (50) connecté à l'actionneur (48) et configuré pour se déplacer par rapport au corps (12),
l'ensemble actionneur (18) étant configuré de sorte que l'actionneur (48) soit disposé à l'intérieur du corps (12) et configuré pour se déplacer par rapport au corps (12) de sorte que l'au moins une surface d'appui entre en contact avec les surfaces de came (41, 43) des bras (14, 16) de manière à faire pivoter les bras (14, 16) d'une configuration non déployée dans laquelle les bras (14, 16) sont sensiblement parallèles à l'axe longitudinal du corps (12) à une configuration déployée dans laquelle les bras (14, 16) sont sensiblement perpendiculaires à l'axe longitudinal du corps (12) ;
les bras (14, 16) étant configurés pour recevoir et loger les processus épineux adjacents lorsqu'ils sont dans la configuration déployée.

2. Écarteur selon la revendication 1, comprenant en outre un élément de retenue (52) configuré pour retenir l'actionneur (48) à l'intérieur du corps (12), l'élément de retenue comprenant une surface filetée intérieure (60) configurée pour se solidariser par filetage à l'arbre fileté (50).

3. Écarteur selon la revendication 2, dans lequel l'élément de retenue comprend deux pinces (72) avec des surfaces filetées intérieures configurées pour recevoir l'arbre fileté (50).

4. Écarteur selon la revendication 3, dans lequel au moins une pince (72) est divisée.

5. Écarteur selon les revendications 3 et 4, dans lequel un écartement est défini entre chaque pince (72) de sorte que l'actionneur (48) puisse passer dans l'écartement.

6. Écarteur selon les revendications 3 à 5, dans lequel les deux pinces (72) sont connectées au niveau de leur extrémité proximale à une extrémité proximale circulaire (70).

7. Écarteur selon les revendications 3 à 6, dans lequel les deux pinces (72) sont flexibles par rapport à l'extrémité proximale circulaire (70).

8. Écarteur selon les revendications 6 et 7, dans lequel l'extrémité proximale circulaire (70) est configurée pour être fixée au corps (12) de sorte que l'actionneur (48) et l'arbre fileté (50) soient connectés mobiles par rapport au corps (12).

9. Écarteur selon les revendications 1 à 8, dans lequel les premier et second bras (14, 16) sont configurés pour pivoter et se déplacer par translation par rapport au corps (12).

10. Écarteur selon les revendications 1 à 9, dans lequel les ouvertures (28) sont configurées de sorte que les premier et second bras (14, 16) soient capables de s'étendre vers l'extérieur à l'opposé du corps.

11. Écarteur selon les revendications 1 à 10, dans lequel les premier et second bras (14, 16) pivotent d'environ 90 degrés dans au moins une configuration déployée dans laquelle les bras (14, 16) sont approximativement transversaux à l'axe longitudinal du corps (12) ;
ladite rotation étant à partir d'une configuration non déployée dans laquelle les bras (14, 16) sont sensiblement parallèles à l'axe longitudinal du corps (12).

12. Écarteur selon les revendications 1 à 11, dans lequel les premier et second bras (14, 16) pivotent d'environ 90 degrés dans au moins une configuration déployée dans laquelle les bras (14, 16) sont approximativement transversaux à l'axe longitudinal du corps (12) ;
ladite rotation étant à partir d'une configuration non déployée dans laquelle les bras (14, 16) sont sensiblement parallèles à l'axe longitudinal du corps (12) ; et
dans lequel, après ladite rotation, au moment d'un autre actionnement, les bras (14, 16) se déplacent par translation par rapport au corps (12) dans une direction sensiblement transversale à l'axe longitudinal du corps (12).

13. Écarteur selon les revendications 1 à 12, dans lequel le passage comprend une surface filetée intérieure configurée pour se solidariser par filetage à l'arbre fileté (50).

14. Écarteur selon les revendications 1 à 13, dans lequel le corps (12) comprend des encoches (34) configurées pour être fixées à un instrument d'amenée d'écarteur, de sorte que l'axe longitudinal de l'instrument d'amenée d'écarteur soit sensiblement aligné avec l'axe longitudinal de l'écarteur (10).

15. Écarteur selon les revendications 1 à 14, dans lequel la rotation de l'arbre fileté (50) dans une direction fait avancer l'actionneur (48) de manière distale pour entrer en contact avec les surfaces de came (41, 43) et déployer les bras (14, 16) dans au moins une configuration déployée, et dans lequel la rotation de l'arbre fileté (50) dans une direction opposée déplace l'actionneur (48) de manière proximale pour permettre aux bras (14, 16) de se plier dans au moins une configuration non déployée ou intermédiaire.

16. Écarteur selon les revendications 1 à 15, dans lequel chaque bras (14, 16) comporte une paire d'extensions (42a, 42b, 44a, 44b) et une selle (46) définissant la configuration sensiblement en forme de U pour loger un processus épineux.
